# EUROPÄISCHE PATENTANMELDUNG

(11) **EP 3 255 608 A1**
(43) Veröffentlichungstag der Anmeldung: **13.12.2017**
(21) Anmeldenummer: 17161766.5
(22) Anmeldetag: 20.03.2017
(51) Int. Cl.: G06T 7/254

(54) **VERFAHREN UND SYSTEM ZUM ERFASSEN EINER LAGEVERÄNDERUNG EINES OBJEKTS**

(71) Anmelder: Siemens Healthcare GmbH, 91052 Erlangen (DE)
(72) Erfinder: Bauer, Christoph, 90491 Nürnberg (DE); Colombo, Vittorio, 90765 Fürth-Poppenreuth (DE)

(57) **Zusammenfassung**

Die Erfindung betrifft ein Verfahren und ein System zum Erfassen einer Lageveränderung eines Objekts (4). Dabei wird ein Diagnosebild (8, 16) des Objekts (4) in einer Scanposition (5) mit einem bildgebenden medizinischen Verfahren erzeugt. Nach dem Erzeugen des Diagnosebildes (8, 16) wird ein Kamerabild (17) des Objekts (4) in einer aktuellen Position aus einer in einer vorgegebenen räumlichen Lagebeziehung zu dem Diagnosebild (8, 16) stehenden Perspektive aufgenommen. Durch einen Abgleich (21) zwischen dem Diagnosebild (8, 16) und dem Kamerabild (17) wird eine Abweichung zwischen der aktuellen Position (14) des Objekts (4) und einer relativ zu der Scanposition (5) definierten Sollposition (5) ermittelt.

## Beschreibung

Die Erfindung betrifft ein Verfahren und ein System zum Erfassen einer Lageveränderung eines Objekts. Ein bevorzugter Anwendungsfall der Erfindung liegt dabei im Bereich medizinischer bildgebender Verfahren und darauf aufbauender weiterführender Maßnahmen, wie beispielsweise einer Vorbereitung strahlentherapeutischer Anwendungen.

In diesem Anwendungsgebiet ist es eine heutzutage übliche Vorgehensweise, anhand eines Diagnosebildes eines Patienten eine Stelle des Patienten zu bestimmen, an welcher beispielsweise eine therapeutische Strahlung auftreffen oder einfallen soll beziehungsweise an der eine Markierung zur späteren Verifizierung einer Lage oder Stellung des Patienten angebracht werden soll. Ebenso kann beispielsweise ein Referenzpunk an dem Patienten, beispielsweise an einem anatomisch stabilen Punkt, entsprechend markiert werden. Diese Stelle wird dann an dem Patienten gekennzeichnet, wobei jedoch eine Lageveränderung des Patienten zwischen der Aufnahme des Diagnosebildes und dem Kennzeichnen zu einem unerkannten Fehler führen kann. Das System AlignRT der Firma Vision RT Ltd. nutzt einen Laserprojektor zum Projizieren eines Gitters auf den Patienten und zwei Kameras zum Erfassen eines Referenzbildes und zum späteren Erfassen eines Vergleichsbildes des Patienten. Durch einen Vergleich dieser Bilder kann aus einer Veränderung des projizierten Gitters eine Lageveränderung des Patienten bestimmt werden. Dies ermöglicht es einem Therapeuten dann, den Patienten neu auszurichten. Ebenso wird heutzutage jedoch oftmals bei einer erkannten oder vermuteten Lageveränderung ein neues Diagnosebild erstellt, in der Hoffnung, dass der Patient sich nicht oder zumindest weniger stark bewegt. Dabei erhöht sich nachteilig eine Dosisbelastung für den Patienten.

Aufgabe der vorliegenden Erfindung ist es, eine Lageveränderung eines Objekts zuverlässig und mit besonders geringem Aufwand zu ermöglichen.

Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen sowie in der Beschreibung und in den Zeichnungen angegeben.

Bei dem erfindungsgemäßen Verfahren zum Erfassen einer Lagebeziehung eines Objekts wird zunächst ein Diagnosebild des Objekts in einer Scanposition mit einem bildgebenden medizinischen Verfahren erzeugt. Nach dem Erzeugen des Diagnosebildes wird ein Kamerabild des Objekts in einer aktuellen Position aufgenommen. Das Kamerabild wird dabei aus einer Perspektive aufgenommen, die in einer vorgegebenen räumlichen Lagebeziehung steht zu dem Diagnosebild und/oder zu einer zum Erfassen des Diagnosebildes - beziehungsweise diesem zugrunde liegender Rohdaten - verwendeten Bilderzeugungseinrichtung. Durch einen Abgleich zwischen dem Diagnosebild und dem Kamerabild wird dann eine Abweichung ermittelt zwischen der aktuellen Position des Objekts und einer relativ zu der Scanposition definierten Sollposition des Objekts.

Bevorzugt kann es sich bei dem Objekt um einen Patienten handeln. Dementsprechend wird die Erfindung im Folgenden anhand dieses Beispiels erläutert. Hierdurch ist die Erfindung jedoch nicht auf eine Anwendung zur Erfassung einer Lageveränderung eines Patienten beschränkt, da als Objekt ein beliebiger Gegenstand dienen beziehungsweise verwendet werden kann.

Das Diagnosebild kann zum Beispiel ein Röntgen-, Computertomographie- oder Magnetresonanztomographiebild oder dergleichen sein. Das Erzeugen des Diagnosebildes kann ein Erfassen oder Aufnehmen des Bildes beziehungsweise diesem zugrunde liegender Roh- oder Bilddaten sowie bedarfsweise eine Datenverarbeitung und/oder ein Bereitstellen des Diagnosebildes umfassen. Die Scanposition ist dabei diejenige reale Position oder Lage des Objekts, in welcher es sich beim Erzeugen des Diagnosebildes, insbesondere bei dem Erfassen der dem Diagnosebild zugrunde liegenden Roh- oder Bilddaten, befindet beziehungsweise befunden hat. Die Scanposition kann also insbesondere relativ zu der zum Erfassen dieser Daten verwendeten Bilderzeugungseinrichtung, beispielsweise also relativ zu einem verwendeten CT- oder MRI-Gerät, definiert sein oder angegeben werden.

Das Kamerabild kann mittels einer Kamera erfasst oder aufgenommen werden. Das Kamerabild kann das Objekt dabei in einem optischen oder sichtbaren Frequenz- beziehungsweise Spektralbereich abbilden, ebenso kann es jedoch möglich sein, für das Kamerabild zusätzlich oder alternativ andere, beispielsweise nicht sichtbare, Frequenz- oder Spektralbereiche zu verwenden. Durch eine geschickte Auswahl kann hier beispielsweise je nach Art oder Beschaffenheit, insbesondere beispielsweise je nach Oberflächenbeschaffenheit oder Reflektivität, des Objekts eine verbesserte Präzision des Kamerabildes und/oder eine verbesserte Abbildung oder Erkennbarkeit des Objektes in dem Kamerabild erreicht werden. Die zum Aufnehmen des Kamerabildes verwendete Kamera kann bevorzugt an der für das Diagnosebild verwendeten Bilderzeugungseinrichtung angeordnet oder festgelegt sein.

Die Kamera kann starr positioniert oder beweglich gehalten sein. In ersterem Fall ist dabei vorteilhaft eine konsistente räumlich Lagebeziehung zwischen der Kamera und der Bilderzeugungseinrichtung sichergestellt. In letzterem Fall kann beispielsweise eine Sensorik oder Erfassungseinrichtung zum Nachverfolgen oder Bestimmen einer jeweiligen Position, Lage oder Stellung der Kamera relativ zu der Bilderzeugungseinrichtung vorgesehen sein. Somit kann sichergestellt werden, dass das Diagnosebild und das Kamerabild in konsistenter Weise zueinander in Beziehung gesetzt werden können. Dies ist insbesondere dann wichtig, wenn das Diagnosebild und das Kamerabild das Objekt aus unterschiedlichen Perspektiven abbilden und/oder wenn die Kamera zum Zeitpunkt des Aufnehmen des Kamerabildes an einer anderen Stelle angeordnet ist, als die Bilderzeugungseinrichtung zum Zeitpunkt des Erzeugens oder Erfassens des Diagnosebildes beziehungsweise der Bilddaten für das Diagnosebild.

Die Sollposition ist eine Position oder Lage des Objekts, welche es zum Zeitpunkt des Aufnehmens des Kamerabildes einnehmen würde, wenn es sich zwischen diesem Zeitpunkt und dem Zeitpunkt des Erfassens der Bilddaten für das Diagnosebild nicht beziehungsweise nicht in unkontrollierter oder ungeplanter, also insbesondere nicht in durch einen Therapeuten oder Techniker nicht vorgegebenen beziehungsweise vorgesehenen, Art und Weise bewegt hätte. Die Sollposition kann dabei mit der Scanposition übereinstimmen. Ebenso ist es jedoch beispielsweise möglich, dass ein Tisch oder eine Liege, worauf der Patient etwa während des CT- oder MRI-Scans liegt, nach diesem Scan in kontrollierter Weise bewegt, verstellt oder verfahren wird. Dies kann beispielsweise ermöglichen, dass der Patient nicht innerhalb einer oftmals als unangenehm drängend empfundenen Röhre des CT- oder MRI-Gerätes verharren muss. In entsprechender Art und Weise kann auch die Bilderzeugungseinrichtung, das heißt also beispielsweise das CT- oder MRI-Gerät, relativ zu dem Patienten bewegt oder verfahren werden. Damit ergibt sich also eine kontrollierte und bekannte räumliche Lagebeziehung zwischen der Scanposition und der Sollposition. Eine Anordnung des Patienten beziehungsweise des Objekts in einer von der Scanposition unterschiedlichen Sollposition kann dabei beispielsweise den Vorteil haben, dass das Kamerabild besonders einfach und aus einer optimalen Perspektive aufgenommen werden kann und/oder das Objekt beziehungsweise der Patient für weitere Maßnahmen oder Behandlungsschritte besonders einfach zugänglich oder erreichbar ist.

Hat sich der Patient zwischen dem Aufnahmezeitpunkten der Bilddaten für das Diagnosebild und dem Aufnahmezeitpunkten des Kamerabildes bewegt, so kann dies durch den vorgesehenen Vergleich oder Abgleich zwischen dem Diagnosebild und dem Kamerabild ermittelt, also erkannt beziehungsweise festgestellt werden. Bei bisherigen Verfahren versucht beispielsweise ein jeweiliger Techniker selbsttätig visuell eine derartige Bewegung, das heißt Lageveränderung, des Patienten festzustellen. Demgegenüber kann es vorliegend bevorzugt vorgesehen sein, dass automatisch ermittelt wird, ob eine solche Abweichung gegeben ist, das heißt ob sich der Patient entsprechend bewegt hat. Dazu kann beispielsweise automatisch eine Differenz beziehungsweise ein Differenzbild zwischen dem Diagnosebild und dem Kamerabild berechnet werden. Hierfür kann insbesondere vorgesehen sein, dass das Diagnosebild und das Kamerabild die gleiche Dimensionalität aufweisen, dass also beispielsweise beide 2D- oder beide 3D-Bilder sind.

In bevorzugter Ausgestaltung der Erfindung kann das Diagnosebild beispielsweise erzeugt werden durch Erfassen von dreidimensionalen Scandaten des Objekts und eine anschließende virtuelle dreidimensionale Rekonstruktion des Objekts beziehungsweise einer Darstellung des Objekts auf Basis der Scandaten. Bevorzugt wird dann auch das Kamerabild als 3D-Bild des Objekts aufgenommen. Für das Erzeugen des dreidimensionalen Diagnosebildes können bekannte Volumenrenderingtechniken (VRT) eingesetzt werden. Zum Aufnehmen des dreidimensionalen Kamerabildes kann beispielsweise eine stereoskopische oder 3D-Kamera verwendet werden. Ebenso kann es aber beispielsweise möglich sein, als Diagnosebild lediglich ein zweidimensionales Schnittbild und als Kamerabild ein herkömmliches zweidimensionales Bild oder Foto zu verwenden.

Zusätzlich oder alternativ zu einer Differenzbildung zwischen dem Diagnosebild und dem Kamerabild kann vorteilhaft eine automatische Bilderkennung verwendet werden, um in dem Diagnosebild und in dem Kamerabild einander entsprechende Punkte oder Regionen zu identifizieren. Da jeweilige durch das Diagnosebild und das Kamerabild erfasste oder abgebildete reale Raumbereiche direkt oder über die bekannte Sollposition in einer bekannten räumlichen Beziehung zueinander stehen, kann dann beispielsweise durch einen Vergleich jeweilige Pixel- oder Voxelkoordinaten einander entsprechender Punkte oder Bereiche in dem Diagnosebild und dem Kamerabild die Abweichung ermittelt werden. Eine Möglichkeit, derartige einander entsprechende Punkte oder Bereiche aufzufinden oder zu identifizieren, kann in der automatischen Verarbeitung des Diagnosebildes und des Kamerabildes durch ein entsprechend trainiertes neuronales Netz gegeben sein.

Das vorliegende Verfahren sieht also vor, dass zum Ermitteln einer gegebenenfalls vorliegenden Abweichung, das heißt einer Positions- oder Lageveränderung des Objekts, das erzeugte Diagnosebild in Verbindung mit dem nach dem Diagnosebild aufgenommenen Kamerabild verwendet wird. Vorteilhaft ist dabei, dass beispielsweise auf einen - bei dem beschriebenen herkömmlichen bekannten Verfahren verwendeten - Projektor zum Projizieren eines Gitters auf dem Patienten verzichtet werden kann. Hierdurch wird nicht nur ein Geräte-, Kalibrierungs- und Kostenaufwand verringert oder eingespart, sondern auch eine mögliche Irritation des Patienten durch das für die Projektion des Gitters verwendete Laserlicht vermieden. Ebenso kann - da die Bilddaten des Diagnosebildes ohnehin erfasst und erzeugt werden und das Objekt notwendigerweise exakt in der Scanposition abbilden - die Lageveränderung ohne zusätzliche Schritte, beispielsweise zum separaten Aufnehmen eines Referenzbildes, ermittelt werden. Da somit also Aufwand zum Aufnehmen des Referenzbildes sowie zum Justieren des Projektors beziehungsweise des projizierten Gitters entfallen kann, kann durch das vorliegende Verfahren vorteilhaft eine Untersuchungs-, Behandlungs- und/oder Vorbereitungszeit des Patienten minimiert werden.

Die Verwendung des Diagnosebildes bietet darüber hinaus den Vorteil, dass nicht nur die Position oder Lage des gesamten Patienten beziehungsweise einer Oberfläche oder Kontur des Patienten zum Ermitteln oder Erfassen der Lageveränderung verwendet werden kann. Vielmehr kann das Diagnosebild dazu verwendet werden, eine tatsächlich und eigentlich relevante Lage oder Lageveränderung eines internen Bereiches des Patienten, beispielsweise eines zu behandelnden Tumors oder dergleichen, ausgewertet und verwendet werden kann. Dabei kann anhand des Diagnosebildes beispielsweise eine Lage dieses relevanten internen Bereiches des Objekts beziehungsweise des Patienten relativ zu der äußeren Oberfläche oder Kontur des Objekts beziehungsweise des Patienten bestimmt werden. Es ist beispielsweise möglich, dass der Patient sich bewegt, dabei aber der relevante interne Bereich in seiner Sollposition verbleibt oder in anderem Maße von dieser abweicht als beispielsweise ein äußerer Oberflächenbereich des Patienten. Anhand des Kamerabildes kann dann beispielsweise ebenfalls nicht nur eine Position oder Lage des gesamten Patienten beziehungsweise einer äußeren Oberfläche oder Kontur des Patienten bestimmt werden, sondern beispielsweise eine Position oder Stellung einer Struktur des Patienten, beispielsweise eines oder mehrerer Knochen oder Gelenke. Hieraus kann beispielsweise ebenfalls auf eine Position oder Lage des internen Bereichs geschlossen werden, da dieser beispielsweise in fester oder bekannter räumlicher Beziehung zu dieser Struktur oder diesen Strukturen angeordnet oder beweglich sein kann. Insgesamt kann somit durch die vorliegende Erfindung die, insbesondere die jeweils relevante, Lageveränderung mit besonders geringerem Geräte- und Zeitaufwand und gleichzeitig verbesserter Genauigkeit oder Präzision ermittelt werden. Dies kann beispielsweise in nachfolgenden Behandlungsschritten zu einem verbesserten Behandlungserfolg beitragen.

Die Lageveränderung, das heißt die Abweichung zwischen der aktuellen Position des Objekts und der Sollposition, kann bevorzugt visuell kenntlich gemacht werden. Hierzu können beispielsweise das Diagnosebild und das Kamerabild mittels einer Anzeigeeinrichtung, beispielsweise eines Bildschirms oder einer Projektionseinrichtung, angezeigt werden. Bevorzugt kann diese Anzeigeeinrichtung an der Bilderzeugungseinrichtung, also beispielsweise an dem CT- oder MRI-Gerät oder zumindest innerhalb desselben Raumes wie die Bilderzeugungseinrichtung angeordnet sein. Dies ermöglicht es dem jeweiligen betreuenden Therapeuten oder Techniker vorteilhaft, das Objekt beziehungsweise den Patienten entsprechend der ermittelten Lageveränderung oder Abweichung besonders zuverlässig neu zu positionieren. In besonders bevorzugter Ausgestaltung kann die Lageveränderung beziehungsweise die Abweichung mittels virtueller oder augmentierter Realität angezeigt werden. So kann beispielsweise ein Head-Mounted-Display (HMD) verwendet werden, mittels welchem beispielsweise die Sollposition des Objekts zusätzlich zu oder in Überlagerung mit der aktuellen Position angezeigt werden kann. Hierdurch kann eine besonders präzise Positionierung des Objekts in der Sollposition ermöglicht werden. Bisherige Verfahren sind im Gegensatz dazu auf eine Abschätzung oder ein Augenmaß des betreuenden Technikers angewiesen, wobei üblicherweise relativ kleine Abweichungen nicht erkannt werden.

Durch die vorliegend vorgeschlagene Visualisierung der Abweichung beziehungsweise der Sollposition kann sichergestellt werden, dass eine Neupositionierung des Objekts dieses tatsächlich in die Sollposition überführt beziehungsweise bewegt. Zusätzlich oder alternativ kann eine akustische Indikation der Abweichung vorgesehen sein. Hierbei kann beispielsweise eine Richtung oder Art, also beispielsweise eine rote rotatorische oder translatorische Differenz, und/oder ein Umfang, das heißt ein Betrag oder eine Größe, der Abweichung beispielsweise durch unterschiedliche Tonhöhen und/oder Lautstärken angegeben werden. Dazu kann beispielsweise ein Steuergerät vorgesehen sein zum Ansteuern des HMD und/oder Lautsprechers in Abhängigkeit von der ermittelten Abweichung. Die Visualisierung und/oder die akustische Angabe der Abweichung können dabei kontinuierlich angepasst werden.

In bevorzugter Ausgestaltung der Erfindung können dazu zusätzlich zu dem einen Kamerabild eine Vielzahl weiterer Kamerabilder, insbesondere eine Videosequenz oder ein kontinuierlicher Videostrom, des Objekts aufgenommen werden. Die Abweichung kann dann, insbesondere in Echtzeit, kontinuierlich durch einen Abgleich zwischen dem Diagnosebild und der Vielzahl weiterer Kamerabilder, insbesondere der Videosequenz, ermittelt werden. Durch die weiteren Kamerabilder beziehungsweise die Videosequenz kann also die aktuelle Position des Objekts kontinuierlich bestimmt beziehungsweise aktualisiert werden. Dazu kann es sich bei der Kamera zur Aufnahme des Kamerabildes um eine Videokamera handeln. Ebenso ist es jedoch möglich, zusätzlich eine oder mehrere Videokameras an der Bilderzeugungseinrichtung oder in bekannter beziehungsweise vorgegebener räumlicher Lagebeziehung relativ zu der Kamera und/oder zu der Bilderzeugungseinrichtung anzuordnen. Hierdurch kann gegebenenfalls die aktuelle Position des Objekts aus unterschiedlichen Blickwinkeln oder Perspektiven erfasst werden, wodurch eine kontinuierliche Erfassung der aktuellen Position des Objekts auch dann möglich ist, wenn beispielsweise ein Blickfeld einer Kamera ganz oder teilweise verdeckt ist.

Die Verwendung virtueller und/oder augmentierter Realität kann vorteilhaft ein direktes, das heißt unmittelbar erkennbares, visuelles Feedback für eine Neupositionierung oder Neuausrichtung des Objekts ermöglichen beziehungsweise bieten. Dabei sind besonders vorteilhaft alle zur korrekten Neupositionierung benötigten Daten in einer gemeinsamen Darstellung oder Ansicht enthalten. Hierdurch kann vorteilhaft beispielsweise die Notwendigkeit für den jeweiligen Techniker vermieden werden, bei der Neupositionierung des Objekts zwischen diesem und einem Bildschirm hin und her zu schauen oder gar den Raum, in dem sich das Objekt befindet, zu verlassen, um die Sollposition abzulesen oder nachzuvollziehen.

In vorteilhafter Ausgestaltung der vorliegenden Erfindung wird die ermittelte Abweichung automatisch ausgeglichen. Dies kann insbesondere durch Bewegen eines das Objekt abstützenden Objektlagers und/oder durch Bewegen einer Markierungseinrichtung - beispielsweise eines Markierungslesers - zum Markieren des Objekts in Abhängigkeit von der ermittelten Abweichung erfolgen. Mit anderen Worten kann also einen Verstellmechanismus, beispielsweise einen Verstell- oder Schrittmotor, zum Verstellen oder Verfahren des Objektlagers und/oder der Markierungseinrichtung vorgesehen sein. Mit anderen Worten wird also als Reaktion auf eine ermittelte oder festgestellte Abweichung, das heißt also eine Lageveränderung des Objekts, automatisch eine Relativbewegung bewirkt. Das Objektlager kann beispielsweise eine Liege oder ein Patiententisch sein, auf dem der Patient während des Erfassens der Bilddaten für das Diagnosebild liegt beziehungsweise angeordnet ist. Die Markierungseinrichtung kann beispielsweise ein als Teil oder an der Bilderzeugungseinrichtung vorgesehener Laser sein, mittels welchem eine bestimmte, beispielsweise in dem Diagnosebild identifizierte Stelle real an dem Patienten angezeigt werden kann. Alternativ oder zusätzlich kann die Markierungseinrichtung beispielsweise in demselben Raum wie das Objektlager angeordnet sein. So können beispielsweise an einer Raumdecke und/oder Wand einer oder mehrere Markierungslaser angeordnet sein. Die Markierungseinrichtung kann dabei insbesondere in bekannter, gegebenenfalls nachvollziehbar veränderlicher, räumlicher Lagebeziehung zu der Bilderzeugungseinrichtung und/oder zu der Kamera beziehungsweise bezogen auf ein gemeinsames Koordinatensystem angeordnet sein.

Die Möglichkeit, sowohl das Objektlager als auch die Markierungseinrichtung, insbesondere unabhängig voneinander, zu bewegen ist dabei besonders vorteilhaft, da hierdurch eine größere Flexibilität erreicht wird. So kann beispielsweise ein möglicher Verfahrweg oder Verstellumfang des Objektlagers und/oder der Markierungseinrichtung begrenzt sein, sodass bei relativ großen Abweichungen diese durch alleinige Verstellung oder Bewegung des Objektlagers oder der Markierungseinrichtung nicht ausgeglichen werden können, da beispielsweise eine Endposition erreicht ist. In einem solchen Fall kann die Abweichung durch eine Verstellung sowohl des Objektlagers als auch der Markierungseinrichtung ausgleichbar sein. Darüber hinaus können das Objektlager und die Markierungseinrichtung ganz oder teilweise unterschiedliche Freiheitsgrade, das heißt beispielsweise verschiedene Richtungen und/oder Verstellmodi, aufweisen. So kann beispielsweise die Markierungseinrichtung auf einer Kreisbahn um eine Längsachse oder -richtung des Objektlagers rotierbar gehalten sein. Hierdurch wird ein Ausgleich der Abweichung in besonders vielen unterschiedlichen Situationen zuverlässig ermöglicht.

Es kann als mögliche Realisierung vorgesehen werden, dass entsprechend, also in Abhängigkeit von der ermittelten Abweichung, das Diagnosebild beziehungsweise eine Darstellung des Diagnosebildes virtuell verschoben oder verstellt wird. Dies kann ebenfalls automatisiert oder durch eine Bedienperson erfolgen. In letzterem Fall kann die von der Bedienperson veranlasste Bewegung oder Verstellung automatisch erfasst werden. In Abhängigkeit von der Bewegung oder Verstellung des Diagnosebildes beziehungsweise von dessen Darstellung kann ein Steuersignal erzeugt werden, welches der Markierungseinrichtung und/oder dem Objektlager zugeführt wird. In Abhängigkeit von oder gemäß dem Steuersignal kann dann die Markierungseinrichtung automatisch in eine korrekte Position verstellt und/oder das Objektlager bewegt werden. Hierdurch ergibt sich effektiv eine Relativbewegung insbesondere zwischen dem Patienten und der Markierungseinrichtung, sodass mittels der Markierungseinrichtung die vorgesehene Stelle an dem Patienten markiert wird beziehungsweise markiert werden kann. Ist eine Aufnahme weiterer Kamerabilder, beispielsweise der Videosequenz oder des Videostromes, vorgesehen, so kann das Objektlager und/oder die Markierungseinrichtung kontinuierlich angesteuert beziehungsweise bewegt werden, um eine kontinuierliche Nachführung des Objektlagers und/oder der Markierungseinrichtung durchzuführen. Es kann also die kontinuierlich ermittelte Abweichung kontinuierlich automatisch ausgeglichen wird.

In vorteilhafter Ausgestaltung der vorliegenden Erfindung wird die Abweichung nur dann automatisch ausgeglichen, wenn sie einen vorgegebenen Schwellenwert überschreitet. Der Schwellenwert kann dabei beispielsweise von einem Therapeuten oder Techniker individuell oder situationsabhängig vorgegeben werden. Ebenso kann der Schwellenwert automatisch ausgewählt oder festgelegt werden. Dazu kann beispielsweise ein Kennfeld oder eine Zuordnungstabelle hinterlegt sein, in der der jeweils zu verwendende Schwellenwert beispielsweise für unterschiedliche Situationen, Therapiestrategien, verwendete Geräte oder Einrichtungen oder dergleichen mehr angegeben ist. Ebenso kann der Schwellenwert beispielsweise durch eine mit den jeweiligen Geräten oder Einrichtungen erzielbare Positionierungsgenauigkeit oder entsprechende Toleranzen vorgegeben oder bestimmt sein. Durch die Verwendung eines derartigen Schwellenwertes kann ein unnötiges Bewegen oder Verstellen vermieden werden. Hierdurch kann vorteilhaft eine für eine entsprechende Bewegung oder Verstellung notwendige Verstellzeit eingespart werden. Zudem kann vorteilhaft beispielsweise ein kontinuierliches Hin- und Herbewegen des Objektlagers und/oder der Markierungseinrichtung vermieden werden, welches sich ohne den Schwellenwert beispielsweise zum Ausgleichen von Atembewegungen des Patienten oder dergleichen mehr einstellen könnte. Hierdurch kann vorteilhaft eine unnötige Belastung und somit ein unnötiger Verschleiß der entsprechenden Geräte, Einrichtungen oder Verstellmechanismen vermieden und ein Patientenkomfort erhöht werden. Beispielhaft kann als Schwellenwert etwa eine Abweichung von 5 mm vorgegeben werden. Der Schwellenwert kann dabei für eine Abweichung in einer Dimension oder Richtung oder für eine resultierende Gesamtabweichung oder Gesamtrichtung vorgegeben werden. Ebenso können für unterschiedliche Richtungen oder Dimensionen beispielsweise unterschiedliche Schwellenwerte vorgegeben werden. Dadurch können vorteilhaft beispielsweise unterschiedliche Richtungscharakteristika, beispielsweise einer therapeutischen Strahlung, berücksichtigt werden.

Zusätzlich oder alternativ zu dem Schwellenwert können beispielsweise irrelevante Teilbereiche des Objekts definiert beziehungsweise entsprechende Definitionen vorgegeben oder erfasst werden. So kann beispielsweise eine Bewegung oder Lageveränderung des Kopfes des Patienten irrelevant, also unerheblich sein für eine vorgesehene Behandlung an der Hüfte oder an einem Bein des Patienten. Ermittelte Abweichungen in irrelevanten Bereichen können dann ignoriert werden. Hierdurch kann vorteilhaft beispielsweise eine unnötige Verstellung vermieden werden, wodurch auch die Gefahr vermieden wird, einen jeweils relevanten Bereich zu bewegen. So kann beispielsweise ein Konflikt vermieden oder gelöst werden, wenn nicht sämtliche Teilbereiche des Patienten in ihre jeweiligen Sollpositionen bewegt werden können.

In vorteilhafter Ausgestaltung der vorliegenden Erfindung wird eine erste virtuelle Markierung in dem Diagnosebild erfasst. Diese erste virtuelle Markierung gibt dabei in einem Koordinatensystem des Diagnosebildes eine vorgegebene Sollstelle für eine reale Markierung an dem Objekt an. Das Kamerabild wird an demselben oder einem entsprechenden Koordinatensystem, welches also auch für das Diagnosebild verwendet wird, ausgerichtet, wobei eine zweite virtuelle Markierung in dem Kamerabild an einer Stelle der ersten virtuellen Markierung in dem Koordinatensystem angezeigt wird. Mit anderen Worten kann also durch die erste und zweite virtuelle Markierung sowohl in dem Diagnosebild als auch in dem Kamerabild bezogen auf das jeweilige Koordinatensystem die gleiche Stelle markiert werden, insbesondere unabhängig von einem Bildinhalt. Es wird also durch die erste virtuelle Markierung nicht notwendigerweise dieselbe Stelle des Patienten markiert oder angezeigt wie durch die zweite virtuelle Markierung, da sich der Patient relativ zu dem Koordinatensystem beziehungsweise etwa relativ zu einem Blickfeld oder Aufnahmebereich der Kamera und/oder der Bilderzeugungseinrichtung bewegt haben kann. Das Koordinatensystem kann beispielsweise seinen Ursprung, also Nullpunkt jeweils im Zentrum des Diagnosebildes beziehungsweise des Kamerabildes oder beispielsweise in einer jeweiligen unteren linken Ecke haben. Das Koordinatensystem korreliert also mit dem jeweiligen Bildausschnitt oder realen Erfassungsbereich.

Kommt es zu einer Lageveränderung des Objekts zwischen den Aufnahmezeitpunkten des Diagnosebildes und des Kamerabildes wird dadurch nicht der jeweilige Bildausschnitt oder Erfassungsbereich und somit auch nicht das Koordinatensystem verschoben, sondern bezogen auf dieses feste Koordinatensystem wird das Objekt in dem Kamerabild gegenüber dem Diagnosebild verschoben dargestellt. In einem solchen Fall sind die erste und zweite virtuelle Markierung also weiterhin bezogen auf das Koordinatensystem an denselben beziehungsweise einander entsprechenden Stellen in beiden Bildern dargestellt, wobei diese Stellen jedoch unterschiedliche Punkte des realen Objektes darstellen oder repräsentieren können. Dementsprechend kann also aus einem Vergleich der beiden Bilder, das heißt des Diagnosebildes und des Kamerabildes, unter Berücksichtigung der virtuellen Markierungen eine eventuelle Lageveränderung des Objekts ermittelt werden.

Alternativ kann es vorgesehen sein, dass das Kamerabild dem Diagnosebild überlagert und dementsprechend die virtuelle Markierung in der Überlagerung beider Bilder, das heißt also in beiden Bildern, angezeigt wird. Durch die Überlagerung zwischen dem Diagnosebild und dem Kamerabild kann unmittelbar und besonders einfach die Abweichung ermittelt beziehungsweise abgelesen werden.

Die erste Alternative, bei der das Diagnosebild und das Kamerabild, insbesondere unabhängig voneinander, beispielsweise auf verschiedenen Bildschirmen, dargestellt werden können, hat den Vorteil, dass eine Erkennbarkeit der jeweiligen Darstellungen nicht beeinträchtigt wird. Dies erlaubt dem jeweiligen Therapeuten oder Techniker also eine besonders einfache, genaue und zuverlässige Einschätzung der Situation beziehungsweise der Abweichung. Die zweite Alternative, bei der die beiden Bilder überlagert werden erlaubt es vorteilhaft, beide Bilder und die Abweichung gemeinsam, das heißt mit einem Blick, insbesondere gleichzeitig, zu erfassen. Während die erste virtuelle Markierung in dem Diagnosebild also eine gewollte oder beabsichtigte Stelle angibt, an welcher das reale Objekt markiert werden soll, zeigt die erste virtuelle Markierung in dem Kamerabild oder die zweite virtuelle Markierung in dem Kamerabild eine tatsächliche Stelle an, auf welche beispielsweise eine Markierungseinrichtung, insbesondere beispielsweise ein Markierungsleser, in der aktuellen Position des Objekts ausgerichtet ist. Diese tatsächliche Stelle oder Markierungsstelle würde in der aktuellen Position also von der Markierungseinrichtung tatsächlich an dem realen Objekt markiert werden, wenn die Abweichung nicht ausgeglichen wird. Ein Ausgleichen der Abweichung kann dadurch erreicht werden, dass die tatsächliche Markierungsstelle mit der Sollstelle in Übereinstimmung gebracht wird - beispielsweise durch Bewegen oder Neuausrichten des Objekts, des Objektlagers und/oder der Markierungseinrichtung.

In weiterer vorteilhafter Ausgestaltung der vorliegenden Erfindung wird das Diagnosebild aus einer zeitlichen Sequenz von Scandaten erzeugt. Die zeitliche Sequenz von Scandaten kann also beispielsweise eine Abfolge von nacheinander aufgenommenen Bildern, also Einzelbildern des Objekts sein, wobei diese Einzelbilder aus der gleichen Perspektive oder beispielsweise aus unterschiedlichen Winkeln aufgenommen werden können. Weiterhin ist es dabei vorgesehen, dass zusätzlich zu dem einen Kamerabild eine Vielzahl weiterer Kamerabilder des Objekts aufgenommen wird. Diese Vielzahl weiterer Kamerabilder können beispielsweise einzelne Frames einer Videosequenz oder eines Videostromes sein. Weiterhin ist es hierbei vorgesehen, dass die Scandaten gemäß ihrer zeitlichen Korrelation mit jeweiligen Aufnahmezeitpunkten der Vielzahl von Kamerabildern jeweils einem der Kamerabilder zugeordnet werden (binning). Hierdurch kann also eine Korrelation oder Zuordnung zwischen den Kamerabildern und den Einzelbildern der Sequenz von Scandaten realisiert werden. Dadurch können beispielsweise Bewegungen oder Lageveränderungen des Patienten berücksichtigt werden, welche während der Aufnahme oder Erfassung der zeitlichen Sequenz von Scandaten auftreten.

Bevorzugt kann dabei durch die Vielzahl der Kamerabilder eine Bewegung oder Lageveränderung des Patienten beziehungsweise einer Oberfläche des Patienten nachverfolgt werden. Aus den Kamerabildern kann so besonders einfach beispielsweise eine Atemkurve, das heißt die durch Atembewegungen verursachte periodische Bewegung des Patienten, ermittelt werden. Die Atembewegung oder Atemkurve des Patienten kann beispielsweise dann besonders relevant sein, wenn durch diese Bewegung sich auch eine räumliche Position oder Lage eines therapeutisch relevanten Bereiches des Patienten, beispielsweise eines internen Tumors oder dergleichen, bewegt oder verändert. Durch die Zuordnung zwischen den Einzelbildern der Sequenz von Scandaten zu den Kamerabildern beziehungsweise der Atemkurve können ein Verständnis der Position, Lage und Bewegung eines solchen Bereiches und dementsprechend auch eine nachfolgende Behandlung beziehungsweise Behandlungsplanung erleichtert und verbessert werden, da beispielsweise eine präzisere Bestrahlung des relevanten Bereiches ermöglicht wird.

Beispielsweise kann anhand der Zuordnung ein optimaler Bestrahlungszeitpunkt in Relation zu der Atemkurve, das heißt also zu der letztlich unvermeidlichen Bewegung des Patienten, bestimmt werden. Dementsprechend kann beispielsweise eine therapeutische Bestrahlung des Patienten mit dessen Atemkurve synchronisiert werden, sodass beispielsweise der relevante Bereich jeweils nur während eines Atemmaximum und/oder eines Atemminimum bestrahlt und andere Bereiche nicht unnötig exponiert werden. Hierdurch kann beispielsweise ein verbessertes Behandlungsergebnis und/oder eine verringerte Dosisbelastung des Patienten erreicht werden. Das erfindungsgemäße Verfahren ermöglicht dies vorteilhaft mit besonders geringem Aufwand, da auch hier lediglich die ohnehin erfassten Scandaten sowie die Kamera verwendet werden müssen. Anders als bei herkömmlichen bekannten Verfahren kann bei dem vorliegend vorgeschlagenen Verfahren also beispielsweise auf zur Erfassung einer Atemkurve vorgesehene Gürtel oder physische Markierungselemente an dem Patienten verzichtet werden. Hierdurch können der Patientenkomfort erhöht und eine Fehlerrate, beispielsweise durch fehlerhafte Bedienung oder Anordnung des Gürtels oder der Markierungselemente, reduziert werden.

Im Gegensatz zu herkömmlichen bekannten Verfahren wird auch beispielsweise kein sonstiges weiteres System benötigt, wodurch ein Geräte-, Installations-, Wartungs-und Datenverarbeitungsaufwandes reduziert werden kann. Andere herkömmliche Verfahren, wie beispielsweise SmartDeviceless 4D von General Electric, nutzen beispielsweise einen Fluoroskopie-Scan der mit dem vorliegend vorgeschlagenen Verfahren ebenfalls entfallen kann, wodurch vorteilhaft eine Strahlungsexposition des Patienten reduziert werden kann. Auch setzt das vorliegende Verfahren im Gegensatz zur Verwendung eines Fluoroskopie-Scans vorteilhaft beispielsweise keine kontinuierliche oder gleichmäßige Atmung des Patienten voraus. Zudem kann vorteilhaft beispielsweise ein Datenimport von dem Gürtel oder dem anderen verwendeten System entfallen. Bei dem vorliegenden Verfahren sind vorteilhaft für den gesamten Scan, das heißt für den Zeitraum der Erfassung der zeitlichen Sequenz von Scandaten, reale Daten verfügbar, sodass keine Extrapolation aus einem limitierten Datensatz von Messwerten notwendig ist, wodurch eine Genauigkeit und Zuverlässigkeit verbessert und eine Fehlerwahrscheinlichkeit reduziert werden kann.

In vorteilhafter Ausgestaltung der vorliegenden Erfindung wird anhand, das heißt auf Basis, des Diagnosebildes und/oder des Kamerabildes eine Position eines mit dem Objekt in Wirkverbindung stehenden Hilfsobjekts bestimmt. Diese bestimmte Position des Hilfsobjekts beziehungsweise entsprechende Positionsdaten werden dann gespeichert. Bei dem Hilfsobjekt kann es sich beispielsweise um eine Lagerungshilfe handeln, durch welche das Objekt abgestützt oder gehalten wird. Durch die Bestimmung dieser Position des Hilfsobjektes und die Speicherung der entsprechenden Positionsdaten, welche die bestimmte Position angeben, wird vorteilhaft eine Reproduzierbarkeit der Position oder Lage des Objekts erleichtert. Die Positionsdaten können die Position des Hilfsobjekts dabei beispielsweise relativ zu der Bilderzeugungseinrichtung, relativ zu der Kamera, relativ zu einem gemeinsamen Koordinatensystem und/oder relativ zu dem Objekt angeben.

In bevorzugter Weiterbildung der Erfindung wird die bestimmte und gespeicherte Position des Hilfsobjekts zum Positionieren des Objekts für eine nachfolgende Untersuchung oder Manipulation des Objekts abgerufen, beispielsweise aus einer entsprechenden Speichereinrichtung. Mit anderen Worten kann also die bestimmte Position beispielsweise in einer Patientenakte oder Patientendatei oder einer sonstigen Datenbank gespeichert werden und steht dann vorteilhaft zu beliebigen späteren Zeitpunkten zur Verfügung. Dadurch beispielsweise eine verbesserte Reproduzierbarkeit bei dem Positionieren des Objekts beispielsweise in der ursprünglich verwendeten Scanposition ermöglicht beziehungsweise erleichtert werden. Dabei kann es vorgesehen sein, dass die Position des Hilfsobjekts automatisch abgerufen und/oder automatisch angezeigt wird, beispielsweise in Form einer visuellen Markierung, insbesondere in einer virtuellen oder augmentierten Realität. Es kann ebenso vorgesehen sein, dass das Hilfsobjekt oder ein funktional entsprechendes zweites Hilfsobjekts automatisch in die abgerufene Position oder eine entsprechende Position - beispielsweise relativ zu dem Patienten - bewegt wird. Insgesamt kann hierdurch eine Fehlerrate beim Positionieren des Objekts verringert werden.

Vorteilhaft kann diese Ausgestaltung des Verfahrens beispielsweise angewendet werden, wenn der Patient, nachdem er im Rahmen des erfindungsgemäßen Verfahrens markiert worden ist, beispielsweise zur Durchführung einer Bestrahlung in einer Position oder Lage angeordnet beziehungsweise ausgerichtet wird beziehungsweise werden soll, welche idealerweise der Scanposition entspricht. Dabei kann die Bestrahlung insbesondere an einem Gerät durchgeführt werden, welches verschieden ist von dem zum Erzeugen des Diagnosebildes verwendeten Gerät. Auch für beispielsweise mehrere zeitlich voneinander beabstandete Bestrahlungsvorgänge kann so zuverlässig dieselbe Position des Objekts reproduziert werden. Dies ermöglicht vorteilhaft eine besonders genaue und konsistente Therapie und somit beispielsweise ein verbessertes Behandlungsergebnis bei gleichzeitig reduzierter Strahlenbelastung. Besonders vorteilhaft ist dabei kein zusätzlicher Geräte- oder Messaufwand notwendig, da auch hier die ohnehin erfassten Daten beziehungsweise Bilder verwendet werden.

Ebenso kann es beispielsweise möglich sein, die abgerufenen Positionsdaten mit einem neuen Kamerabild, welches an dem jeweils aktuell verwendeten Gerät, beispielsweise an dem Bestrahlungsgerät, aufgenommen wird, zu korrelieren beziehungsweise abzugleichen.

Ein erfindungsgemäßes System zum Erfassen einer Lageveränderung eines Objekts umfasst ein Steuergerät, eine Bilderzeugungseinrichtung, eine Kamera und eine Datenverarbeitungseinrichtung. Die Kamera ist dabei in einer vorgegebenen räumlichen Lagebeziehung zu der Bilderzeugungseinrichtung angeordnet. Das Steuergerät ist dazu eingerichtet, die Bilderzeugungseinrichtung anzusteuern zum Erzeugen eines Diagnosebildes des Objekts in einer Scanposition mit einem bildgebenden medizinischen Verfahren. Das Steuergerät ist weiterhin dazu eingerichtet, die Kamera anzusteuern zum Aufnehmen eines Kamerabildes des Objekts in einer aktuellen Position nach dem Erzeugen des Diagnosebildes. Das Steuergerät ist weiterhin dazu eingerichtet, die Datenverarbeitungseinrichtung anzusteuern zum Ermitteln einer Abweichung zwischen der aktuellen Position des Objekts und einer relativ zu der Scanposition definierten Sollposition durch einen Abgleich zwischen dem Diagnosebild und dem Kamerabild.

Die bisher und im Folgenden angegebenen Eigenschaften und Weiterbildungen des erfindungsgemäßen Verfahrens sowie die entsprechenden Vorteile sind jeweils sinngemäß auf das erfindungsgemäße System und/oder die zur Durchführung des erfindungsgemäßen Verfahrens verwendeten oder verwendbaren Bauteile und Einrichtungen übertragbar und umgekehrt. Es gehören also zu der Erfindung auch solche Weiterbildungen des erfindungsgemäßen Verfahrens und des erfindungsgemäßen Systems, welche Ausgestaltungen aufweisen, die hier nicht explizit in der jeweiligen Kombination beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen und dabei zeigen:
- FIG 1: eine schematische Perspektivansicht eines bildgebenden medizinischen Geräts und eines damit abzubildenden Patienten in einer Scanposition;
- FIG 2: eine schematische Darstellung einer mittels des medizinischen bildgebenden Geräts erzeugten Scandarstellung des Patienten mit einer virtuellen Markierung;
- FIG 3: die Darstellung aus FIG 1, wobei sich der Patient nach einem Scan mit dem medizinischen bildgebenden Gerät in einer aktuellen Position befindet und ein Kamerabild des Patienten erfasst wird; und
- FIG 4: eine schematische Gegenüberstellung eines mit dem medizinischen Gerät erzeugten Diagnosebildes und eines Kamerabildes des Patienten jeweils mit virtuellen Markierungen.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den FIG sind gleiche, funktionsgleiche oder einander entsprechende Elemente jeweils mit den gleichen Bezugszeichen gekennzeichnet.

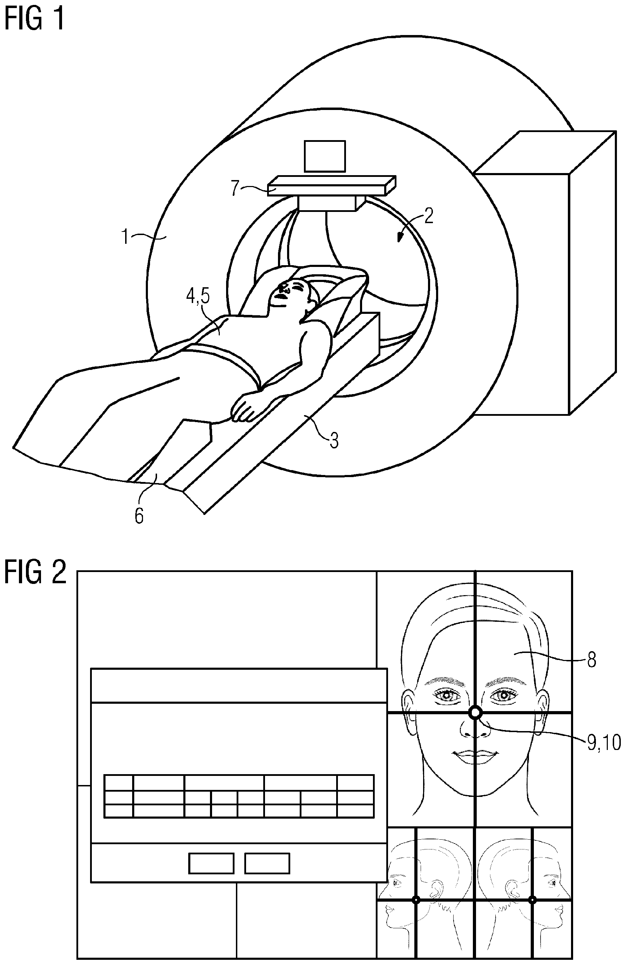

FIG 1 zeigt eine schematische und ausschnittweise Perspektivansicht eines Computertomographen 1, welcher ringförmig einen Scanbereich 2 umgibt. In diesen Scanbereich 2 und aus diesem Scanbereich 2 heraus kann eine hier ebenfalls dargestellte Patientenliege 3 automatisch bewegt werden. Vorliegend befindet sich auf der Patientenliege 3 ein Patient 4 in einer Scanposition 5. Um den Patienten 4 in dieser Scanposition 5 zu lagern beziehungsweise zu unterstützen, ist zwischen dem Patienten 4 und der Patientenliege 3 bereichsweise eine den Patienten 4 abstützenden Lagerungshilfe 6 angeordnet. Weiterhin ist vorliegend oberhalb des Scanbereich 2 an dem Computertomographen 1 eine Kamera 7 angeordnet, bei der es sich vorliegend um eine stereoskopische beziehungsweise 3D-Kamera handelt. Mittels der Kamera 7 können also dreidimensionale Bilder, insbesondere des Patienten 4, aufgenommen werden.

Ausgehend von der in FIG 1 dargestellten Situation wird im Folgenden nun ein Verfahren zum Erfassen einer Lageveränderung des Patienten 4 erläutert.

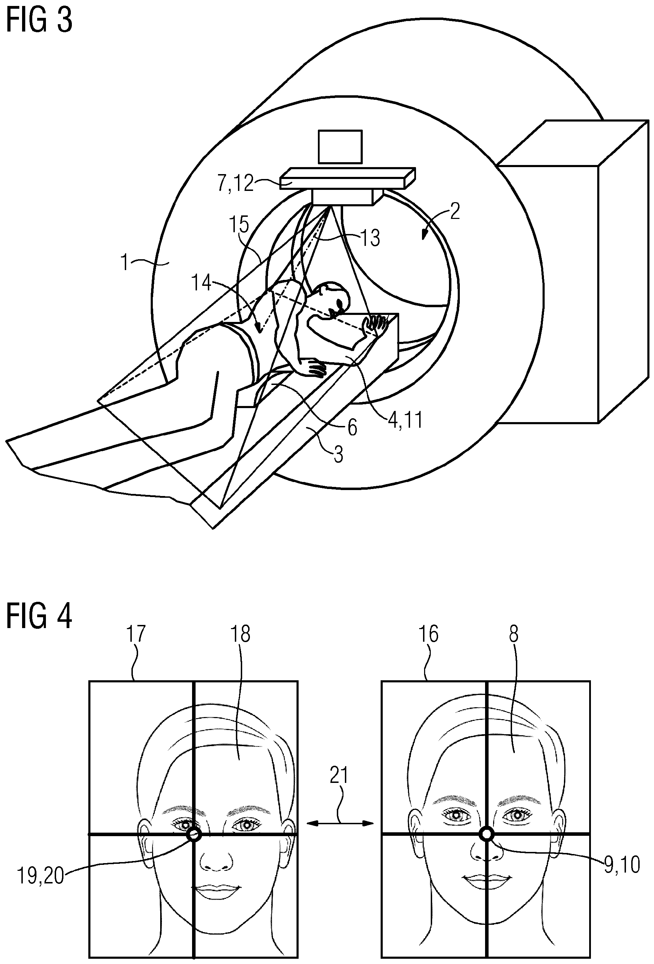

Nachdem der Patient 4 in der Scanposition 5 angeordnet worden ist, wird die Patientenliege 3 mit dem darauf befindlichen Patienten 4 ganz oder teilweise in den Scanbereich 5 bewegt. Mittels des Computertomographen 1 werden dann Scandaten des Patienten 4 erfasst beziehungsweise aufgenommen oder erzeugt. Aus diesem Scandaten kann dann eine virtuelle dreidimensionale Repräsentation des Patienten 4 beziehungsweise eines mit dem Computertomographen 1 erfassten Teilbereiches des Patienten 4 rekonstruiert werden. Dazu kann eine hier nicht dargestellte Datenverarbeitungseinrichtung, beispielsweise ein Computer, verwendet werden. Der Computertomograph 1 beziehungsweise der Computertomograph 1 zusammen mit der Datenverarbeitungseinrichtung bilden also eine Bilderzeugungseinrichtung zum Erzeugen eines Diagnosebildes 16 (vergleiche FIG 4) des Patienten 4.

Aus den mittels des Computertomographen 1 aufgenommenen Scandaten wird also automatisch eine schematisch in FIG 2 dargestellte Scandarstellung 8 des Patienten 4 erzeugt, welche beispielsweise durch einen jeweiligen Therapeuten ausgewertet werden kann. Im vorliegenden Beispiel ist es vorgesehen, dass der Patient 4 eine Bestrahlungsbehandlung erhalten soll. Dazu gibt der Therapeut in der Scandarstellung 8 eine erste virtuelle Markierung 9 vor, durch welche eine Sollstelle 10 markiert wird. Die Sollstelle 10 kann hier diejenige Stelle an oder in dem Patienten 4 angeben, an welcher die Bestrahlungsbehandlung ihre Wirkung entfalten soll. Die Sollstelle 10 kann aber alternativ oder zusätzlich Sollstelle 10 eine Stelle sein, an der eine Markierung zur späteren Verifizierung einer Lage oder Stellung des Patienten 4 angebracht werden soll. Ebenso kann die Sollstelle 10 ein anatomisch stabiler Referenzpunkt auf dem Patienten - beispielsweise an oder auf einem Beckenkamm, sein, welcher sich in einer bekannten räumlichen Relation zu einer tatsächlich zu bestrahlenden oder zu markierenden Stelle befindet.

Da die Bestrahlungsbehandlung nicht mittels des Computertomographen 1, sondern beispielsweise mittels eines Linearbeschleunigers oder einer Protonentherapieeinrichtung durchgeführt wird und zwischen der Aufnahme der Scandaten mittels des Computertomographen 1 und der Bestrahlungsbehandlung eine erhebliche Zeitspanne vergehen kann, ist es notwendig, sicherzustellen, dass die vorgesehene Sollstelle zuverlässig und reproduzierbar wiederauffindbar ist. Dazu ist es vorgesehen, dass an dem Patienten 4 eine entsprechende permanente Markierung, beispielsweise ein Tattoo oder dergleichen angebracht wird. Dabei besteht die Schwierigkeit, oder Problematik, sicherzustellen, dass dieses Tattoo tatsächlich die vorgesehene Sollstelle 10 markiert.

FIG 3 zeigt schematisch eine Situation ähnlich zu der bereits in FIG 1 gezeigten Situation. In der in FIG 3 gezeigten Situation ist die Patientenliege 3 nach dem Aufnehmen der Scandaten wieder aus dem Scanbereich 2 herausgefahren. Da zwischen den in FIG 1 und FIG 3 gezeigten Situationen eine gewisse Zeitspanne verstrichen ist, hat sich der Patient in dieser Zwischenzeit bewegt und befindet sich in FIG 3 nunmehr in einer aktuellen Position 11, welche insbesondere von der Scanposition 5 verschieden sein kann.

Um diese Problematik zu lösen, das der Patient nicht notwendigerweise bewegungslos verharrt, ist es vorliegend vorgesehen, dass mittels einer, insbesondere durch die Datenverarbeitungseinrichtung oder ein mit dieser verbundenes Steuergerät, gesteuerte Markierungseinrichtung eine zu markierende Stelle an dem Patienten automatisch angezeigt wird. Vorliegend ist dazu an der Kamera 7 ein Markierungslaser 12 vorgesehen, welcher einen Laserstrahl 13 aussendet. Der Markierungslaser 12 ist dabei so angesteuert, dass eine durch den Laserstrahl 13 an dem Patienten 4 angezeigte Markierungsstelle 14 gerade die in der Scandarstellung virtuell vorgegebene Sollstelle 10 anzeigt oder markiert, wenn sich der Patient nicht bewegt hat. Dabei besteht jedoch die weitere Problematik, dass zwischen der Aufnahme der Scandaten, durch die rechen- und zeitaufwendige Erzeugung der Scandarstellung 8 und die notwendige Auswertung durch den Therapeuten eine signifikante Zeitspanne - beispielsweise in 5 bis 20 Minuten - vergehen kann. Um dem Patienten 4 diese Wartezeit komfortabler zu gestalten, wird nach dem Aufnehmen der Scandaten die Patientenliege 3 wieder aus dem Scanbereich 2 herausgefahren. Während dieser Wartezeit kann sich der Patient 4 bewegen, also eine Lageveränderung vornehmen. Dementsprechend kann sich die aktuelle Position 11 des Patienten 4 von der Scanposition 5 unterscheiden.

Um dieser weiteren Problematik zu begegnen, ist es vorliegend vorgesehen, dass mittels der Kamera 7, deren Aufnahmebereich 15 hier schematisch angedeutet ist, vor der Anbringung des Tattoos ein Kamerabild 17 (vergleiche FIG 4) aufgenommen wird. Da die Kamera 7 in einer bekannten räumlichen Lagebeziehung zu dem Computertomographen 1 steht, also angeordnet ist, besteht auch eine bekannte Lagebeziehung oder Relation zwischen dem Aufnahmebereich 15 beziehungsweise dem Kamerabild 17 und dem Diagnosebild 16.

FIG 4 zeigt eine schematische Gegenüberstellung des Diagnosebildes 16 mit der Scandarstellung 8 und der ersten virtuellen Markierung 9 einerseits und des Kamerabildes 17 mit einer Kameradarstellung 18 des Patienten 4 in der aktuellen Position 11. Aufgrund der bekannten Relation zwischen dem Diagnosebild 16 und dem Kamerabild 17 kann in dem Kamerabild 17 eine zweite virtuelle Markierung 19 angezeigt werden, deren Position innerhalb des Kamerabildes 17 der Position der ersten virtuellen Markierung 9 in dem Diagnosebild 16 entspricht. Die zweite virtuelle Markierung 19 zeigt darüber hinaus die Markierungsstelle 14 an, auf die der Markierungslaser 12 beziehungsweise der Laserstrahl 13 ausgerichtet ist. Hat sich der Patient 4 zwischen dem Zeitpunkt der Aufnahme der Scandaten und dem Aufnahmezeitpunkt des Kamerabildes 17 bewegt, so entspricht eine durch die zweite virtuelle Markierung 19 markierte Stelle 20 in der Kameradarstellung 18 nicht mehr der Sollstelle 10 bezogen auf den realen Patienten 4.

Diese Abweichung zwischen der in dem Kamerabild 17 markierten Stelle 20 und der Sollstelle 10 wird durch einen schematisch angedeuteten Abgleich 21 zwischen dem Diagnosebild 16 und dem Kamerabild 17 ermittelt. Hierzu kann beispielsweise eine Differenz zwischen dem Diagnosebild 16 und dem Kamerabild 17 berechnet werden. Ist die so ermittelte Abweichung größer als null, so ist damit die Lageveränderung des Patienten zwischen der Scanposition 5 und der aktuellen Position 11 erfasst beziehungsweise ermittelt. Um die Abweichung auszugleichen, kann beispielsweise die Patientenliege 3 automatisch bewegt oder verstellt werden und/oder der Markierungslaser 12 kann automatisch neu ausgerichtet werden. Dadurch kann sichergestellt werden, dass die in dem Kamerabild 17 markierte Stelle 20 dieselbe Stelle des Patienten 4 anzeigt oder markiert, wie die erste virtuelle Markierung 9 in dem Diagnosebild 16. Das bedeutet, dass dann also die reale Markierungsstelle 14 auf dem Patienten 4 der durch die erste virtuelle Markierung 9 vorgegebenen Sollstelle 10 entspricht.

Insgesamt kann so mit besonders geringem Aufwand eine Lageveränderung eines Objekts, das heißt hier des Patienten 4, zuverlässig erfasst und automatisch ausgeglichen werden.

## Patentansprüche

1. Verfahren zum Erfassen einer Lageveränderung eines Objekts, mit den Verfahrensschritten
- Erzeugen eines Diagnosebildes (8, 16) des Objekts (4) in einer Scanposition (5) mit einem bildgebenden medizinischen Verfahren,
- Aufnehmen eines Kamerabildes (17) des Objekts (4) in einer aktuellen Position (11) aus einer in einer vorgegebenen räumlichen Lagebeziehung zu dem Diagnosebild (8, 16) stehenden Perspektive nach dem Erzeugen des Diagnosebildes(8, 16), und
- Ermitteln einer Abweichung zwischen der aktuellen Position (11) des Objekts (4) und einer relativ zu der Scanposition (5) definierten Sollposition durch einen Abgleich (21) zwischen dem Diagnosebild (16) und dem Kamerabild (17).

2. Verfahren nach Anspruch 1, **dadurch gekennzeichnet, dass**
- das Diagnosebild (8, 16) erzeugt wird durch Erfassen von dreidimensionalen Scandaten des Objekts (4) und eine virtuelle dreidimensionale Rekonstruktion (8) auf Basis der Scandaten, und
- das Kamerabild (17) als 3D-Bild des Objekts (4) aufgenommen wird.

3. Verfahren nach einem der vorhergehenden Ansprüche, **dadurch gekennzeichnet, dass** zusätzlich zu dem einen Kamerabild (17) eine Vielzahl weiterer Kamerabilder, insbesondere eine Videosequenz, des Objekts (4) aufgenommen und die Abweichung kontinuierlich durch einen Abgleich zwischen dem Diagnosebild (8, 16) und der Vielzahl weiterer Kamerabilder, insbesondere der Videosequenz, ermittelt wird.

4. Verfahren nach einem der vorhergehenden Ansprüche, **dadurch gekennzeichnet, dass** die ermittelte Abweichung automatisch ausgeglichen wird, insbesondere durch Bewegen eines das Objekt (4) abstützenden Objektlagers (3) und/oder durch Bewegen einer Markierungseinrichtung (12) zum Markieren des Objekts (4) in Abhängigkeit von der ermittelten Abweichung.

5. Verfahren nach Anspruch 4, **dadurch gekennzeichnet, dass** die Abweichung nur dann automatisch ausgeglichen wird, wenn sie einen vorgegebenen Schwellenwert überschreitet.

6. Verfahren nach einem der vorhergehenden Ansprüche, **dadurch gekennzeichnet, dass**
- eine erste virtuelle Markierung (9) in dem Diagnosebild (8, 16) erfasst wird, welche in einem Koordinatensystem des Diagnosebildes (8, 16) eine vorgegebene Sollstelle (10) für eine reale Markierung (14) an dem Objekt (4) angibt, und
- das Kamerabild (17) an demselben oder einem entsprechenden Koordinatensystem ausgerichtet wird, wobei
- eine zweite virtuelle Markierung (19) angezeigt wird an einer Stelle (10) der ersten virtuellen Markierung (9) in dem Koordinatensystem oder wobei
- das Kamerabild (17) dem Diagnosebild (8, 16) überlagert und die erste virtuelle Markierung (9) in der Überlagerung beider Bilder (16, 17) angezeigt wird.

7. Verfahren nach einem der vorhergehenden Ansprüche, **dadurch gekennzeichnet, dass**
- das Diagnosebild (8, 16) aus einer zeitlichen Sequenz von Scandaten erzeugt wird,
- zusätzlich zu dem einen Kamerabild (17) eine Vielzahl weiterer Kamerabilder des Objekts (4) aufgenommen wird, und
- die Scandaten gemäß ihrer zeitlichen Korrelation mit jeweiligen Aufnahmezeitpunkten der Vielzahl von Kamerabildern jeweils einem der Kamerabilder zugeordnet werden.

8. Verfahren nach einem der vorhergehenden Ansprüche, **dadurch gekennzeichnet, dass** anhand des Diagnosebildes (8, 16) und/oder des Kamerabildes (17) eine Position eines mit dem Objekt (4) in Wirkverbindung stehenden Hilfsobjekts (6), insbesondere einer Lagerungshilfe (6), bestimmt wird und entsprechende Positionsdaten gespeichert werden.

9. Verfahren nach Anspruch 8, **dadurch gekennzeichnet, dass** die bestimmte und gespeicherte Position des Hilfsobjekts (6) zum Positionieren des nachfolgende Untersuchung oder Manipulation des Objekts (4) abgerufen wird.

10. System zum Erfassen einer Lageveränderung eines Objekts (4), wobei das System ein Steuergerät, eine Bilderzeugungseinrichtung (1), eine in einer vorgegebenen räumlichen Lagebeziehung zu der Bilderzeugungseinrichtung (1) angeordnete Kamera (7) und eine Datenverarbeitungseinrichtung umfasst, wobei das Steuergerät eingerichtet dazu ist,
- die Bilderzeugungseinrichtung anzusteuern zum Erzeugen eines Diagnosebildes (8, 16) des Objekt (4) in einer Scanposition (5) mit einem bildgebenden medizinischen Verfahren,
und
- die Kamera (7) anzusteuern zum Aufnehmen eines Kamerabildes (17) des Objekts (4) in einer aktuellen Position (11) nach dem Erzeugen des Diagnosebildes (8, 16), und
- die Datenverarbeitungseinrichtung anzusteuern zum Ermitteln einer Abweichung zwischen der aktuellen Position (11) des Objekts (4) und einer relativ zu der Scanposition (5) definierten Sollposition durch einen Abgleich (21) zwischen dem Diagnosebild (8, 16) und dem Kamerabild (17).

## Geänderte Patentansprüche

### Geänderte Patentansprüche gemäss Regel 137(2) EPÜ.

1. Verfahren zum Erfassen einer Lageveränderung eines Objekts, mit den Verfahrensschritten
- Erzeugen eines Diagnosebildes (8, 16) des Objekts (4) in einer Scanposition (5) mit einem bildgebenden medizinischen Verfahren,
- Aufnehmen einer Vielzahl von Kamerabildern (17) des Objekts (4) in einer jeweils aktuellen Position (11) aus einer in einer vorgegebenen räumlichen Lagebeziehung zu dem Diagnosebild (8, 16) stehenden Perspektive nach dem Erzeugen des Diagnosebildes(8, 16),
- kontinuierliches Ermitteln einer Abweichung zwischen der jeweils aktuellen Position (11) des Objekts (4) und einer relativ zu der Scanposition (5) definierten Sollposition durch einen kontinuierlichen Abgleich (21) zwischen dem Diagnosebild (16) und den Kamerabildern (17), und
- kontinuierlich angepasstes Visualisieren der Sollposition und der jeweils aktuellen Position (11) des Objekts (4) sowie der kontinuierlich ermittelten Abweichung zwischen diesen in einer augmentierten Realität mittels eines Head-Mounted Displays.

2. Verfahren nach Anspruch 1, **dadurch gekennzeichnet, dass**
- das Diagnosebild (8, 16) erzeugt wird durch Erfassen von dreidimensionalen Scandaten des Objekts (4) und eine virtuelle dreidimensionale Rekonstruktion (8) auf Basis der Scandaten, und
- das Kamerabild (17) als 3D-Bild des Objekts (4) aufgenommen wird.

3. Verfahren nach einem der vorhergehenden Ansprüche, **dadurch gekennzeichnet, dass** die Vielzahl von Kamerabildern (17) des Objekts (4) als Videosequenz aufgenommen wird.

4. Verfahren nach einem der vorhergehenden Ansprüche, **dadurch gekennzeichnet, dass** die ermittelte Abweichung automatisch ausgeglichen wird, insbesondere durch Bewegen eines das Objekt (4) abstützenden Objektlagers (3) und/oder durch Bewegen einer Markierungseinrichtung (12) zum Markieren des Objekts (4) in Abhängigkeit von der ermittelten Abweichung.

5. Verfahren nach Anspruch 4, **dadurch gekennzeichnet, dass** die Abweichung nur dann automatisch ausgeglichen wird, wenn sie einen vorgegebenen Schwellenwert überschreitet.

6. Verfahren nach einem der vorhergehenden Ansprüche, **dadurch gekennzeichnet, dass**
- eine erste virtuelle Markierung (9) in dem Diagnosebild (8, 16) erfasst wird, welche in einem Koordinatensystem des Diagnosebildes (8, 16) eine vorgegebene Sollstelle (10) für eine reale Markierung (14) an dem Objekt (4) angibt, und
- das Kamerabild (17) an demselben oder einem entsprechenden Koordinatensystem ausgerichtet wird, wobei
- eine zweite virtuelle Markierung (19) angezeigt wird an einer Stelle (10) der ersten virtuellen Markierung (9) in dem Koordinatensystem oder wobei
- das Kamerabild (17) dem Diagnosebild (8, 16) überlagert und die erste virtuelle Markierung (9) in der Überlagerung beider Bilder (16, 17) angezeigt wird.

7. Verfahren nach einem der vorhergehenden Ansprüche, **dadurch gekennzeichnet, dass**
- das Diagnosebild (8, 16) aus einer zeitlichen Sequenz von Scandaten erzeugt wird,
- zusätzlich zu dem einen Kamerabild (17) eine Vielzahl weiterer Kamerabilder des Objekts (4) aufgenommen wird, und
- die Scandaten gemäß ihrer zeitlichen Korrelation mit jeweiligen Aufnahmezeitpunkten der Vielzahl von Kamerabildern jeweils einem der Kamerabilder zugeordnet werden.

8. Verfahren nach einem der vorhergehenden Ansprüche, **dadurch gekennzeichnet, dass** anhand des Diagnosebildes (8, 16) und/oder des Kamerabildes (17) eine Position eines mit dem Objekt (4) in Wirkverbindung stehenden Hilfsobjekts (6), insbesondere einer Lagerungshilfe (6), bestimmt wird und entsprechende Positionsdaten gespeichert werden.

9. Verfahren nach Anspruch 8, **dadurch gekennzeichnet, dass** die bestimmte und gespeicherte Position des Hilfsobjekts (6) zum Positionieren des Objekts (4) für eine nachfolgende Untersuchung oder Manipulation des Objekts (4) abgerufen wird.

10. System zum Erfassen einer Lageveränderung eines Objekts (4), wobei das System ein Steuergerät, eine Bilderzeugungseinrichtung (1), eine in einer vorgegebenen räumlichen Lagebeziehung zu der Bilderzeugungseinrichtung (1) angeordnete Kamera (7), eine Datenverarbeitungseinrichtung und ein Head-Mounted Display umfasst, wobei das Steuergerät eingerichtet dazu ist,
- die Bilderzeugungseinrichtung anzusteuern zum Erzeugen eines Diagnosebildes (8, 16) des Objekt (4) in einer Scanposition (5) mit einem bildgebenden medizinischen Verfahren, und
- die Kamera (7) anzusteuern zum Aufnehmen einer Vielzahl von Kamerabildern (17) des Objekts (4) in einer jeweils aktuellen Position (11) nach dem Erzeugen des Diagnosebildes (8, 16),
- die Datenverarbeitungseinrichtung anzusteuern zum kontinuierlichen Ermitteln einer Abweichung zwischen der jeweils aktuellen Position (11) des Objekts (4) und einer relativ zu der Scanposition (5) definierten Sollposition durch einen kontinuierlichen Abgleich (21) zwischen dem Diagnosebild (8, 16) und den Kamerabildern (17) und
- das Head-Mounted Display anzusteuern zum kontinuierlich angepassten Visualisieren der Sollposition und der jeweils aktuellen Position (11) des Objekts (4) sowie der kontinuierlich ermittelten Abweichung zwischen diesen in einer augmentierten Realität mittels des Head-Mounted Displays.
